(19) Europäisches Patentamt
European Patent Office
Office européen des brevets

(11) **EP 4 249 934 A1**

(12) **EUROPÄISCHE PATENTANMELDUNG**

(43) Veröffentlichungstag:
**27.09.2023   Patentblatt 2023/39**

(21) Anmeldenummer: **22164397.6**

(22) Anmeldetag: **25.03.2022**

(51) Internationale Patentklassifikation (IPC):
**G01R 33/24** (2006.01)        **G01R 33/56** (2006.01)
**G01R 33/565** (2006.01)

(52) Gemeinsame Patentklassifikation (CPC):
**G01R 33/246; G01R 33/243; G01R 33/5608;
G01R 33/56563; G01R 33/5659**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH
91052 Erlangen (DE)**

(72) Erfinder: **Liebig, Patrick
91052 Erlangen (DE)**

(54) **TRAINIERTE FUNKTION ZUR BEREITSTELLUNG VON MAGNETFELDDATEN UND IHRE ANWENDUNG**

(57)    Die Erfindung betrifft ein Verfahren zum Erzeugen einer optimierten trainierten Funktion zur Bereitstellung von Magnetfelddaten, ein Verfahren zum Bereitstellen von Magnetfelddaten mittels einer trainierten Funktion, eine Systemsteuereinheit, eine Magnetresonanzvorrichtung und ein Computerprogrammprodukt.

Das Verfahren zum Bereitstellen von Magnetfelddaten umfasst ein Empfangen von Bilddaten als Eingabedaten der trainierten Funktion, ein Anwenden der trainierten Funktion auf die Bilddaten, wobei die trainierte Funktion basierend auf einer Datentreue von anhand der Magnetfelddaten korrigierten Bilddaten und zumindest einer Annahme über zumindest eine Eigenschaft der Magnetfelddaten trainiert ist, und eine Bereitstellung der Magnetfelddaten als Ausgabedaten der trainierten Funktion.

FIG 3

**Beschreibung**

Die Erfindung betrifft ein Verfahren zum Erzeugen einer optimierten trainierten Funktion zur Bereitstellung von Magnetfelddaten, ein Verfahren zum Bereitstellen von Magnetfelddaten mittels einer trainierten Funktion, eine Systemsteuereinheit, eine Magnetresonanzvorrichtung und ein Computerprogrammprodukt.

In der Medizintechnik zeichnet sich die Bildgebung mittels Magnetresonanz (MR), auch Magnetresonanztomographie (MRT, engl. Magnetic Resonance Imaging, MRI) genannt, durch hohe Weichteilkontraste aus. Hierbei wird ein Untersuchungsobjekt, insbesondere ein Patient, in einem Untersuchungsraum einer Magnetresonanzvorrichtung positioniert. Während einer Magnetresonanzmessung werden üblicherweise mit Hilfe einer Sendespulenanordnung der Magnetresonanzvorrichtung hochfrequente (HF) Sendepulse in das Untersuchungsobjekt eingestrahlt. Ferner werden mit Hilfe einer Gradientenspuleneinheit der Magnetresonanzvorrichtung Gradientenpulse ausgegeben, wodurch temporäre Magnetfeldgradienten im Untersuchungsraum erzeugt werden. Durch die erzeugten Pulse werden im Patienten ortskodierte Magnetresonanzsignale angeregt und ausgelöst. Die ausgelösten Magnetresonanzsignale werden von einer Empfangsspulenanordnung der Magnetresonanzvorrichtung empfangen und zur Rekonstruktion von Magnetresonanzabbildungen verwendet.

Durch einen HF-Sendepuls wird ein magnetisches Wechselfeld, ein sogenanntes $B1^+$-Feld, in einem Untersuchungsbereich erzeugt, in dem sich der Patient während der Magnetresonanzmessung befindet. Das $B1^+$-Feld stellt also eine Verteilung des Sendefeldes dar. Dagegen beschreibt ein $B1^-$-Feld eine Verteilung eines Empfangsfeldes dar. Sind die Sendespulenanordnung und die Empfangsspulenanordnung identisch, kann oftmals von einem im Wesentlichen gleichförmigen $B1^+$-Feld und $B1^-$-Feld ausgegangen werden. Das effektive Sende-Empfangs-Feld kann auch als B1-Feld bezeichnet werden.

In dem Untersuchungsbereich wird zudem ein starkes statisches Hauptmagnetfeld, das B0-Feld, erzeugt. Auch dieses sollte möglichst homogen sein, um Artefakte in den Magnetresonanzabbildungen zu vermeiden. In der Realität weist das Hauptmagnetfeld jedoch üblicherweise an manchen Stellen Inhomogenitäten auf. Unter anderem um Inhomogenitäten des Hauptmagnetfeldes kompensieren, etwa mittels Shim-Spulen, zu können, ist auch die Kenntnis des B0-Feldes, also der räumlichen Verteilung des statischen Magnetfelds, von Vorteil.

Die Ermittlung von Magnetfelddaten, die beispielsweise das B1-Feld und/oder das B0-Feld beschreiben, ist jedoch mit üblichen Verfahren oftmals zeitaufwändig und/oder ungenau. Als Aufgabe der vorliegenden Erfindung kann angesehen werden, ein vorzugsweise schnelles und/oder robustes Verfahren zum Bereitstellen von Magnetfelddaten anzugeben. Insbesondere kann als Aufgabe angesehen werden, Mittel bereitzustellen, mit denen ein solches Verfahren durchgeführt werden kann.

Die Aufgabe wird durch die Merkmale der unabhängigen Ansprüche gelöst. Vorteilhafte Ausgestaltungen sind in den abhängigen Ansprüchen beschrieben.

Demnach wird ein computerimplementiertes Verfahren zum Erzeugen einer optimierten trainierten Funktion, OTF, zur Bereitstellung von Magnetfelddaten vorgeschlagen. Das Verfahren umfasst die Schritte:

    a) Bereitstellen von Trainings-Bilddaten, TBD,
    b) Bereitstellen anhand der TDB erzeugter korrigierter Trainings-Bilddaten, KTBD,
    c) Bereitstellen einer vorläufigen trainierten Funktion, VTF,
    d) Erzeugen vorläufiger Magnetfelddaten, VMD, durch Anwenden der TBD auf die VTF,
    e) Erzeugen von vorläufigen korrigierten Bilddaten, VKBD, anhand der VMD und der TBD,
    f) Ermitteln einer Datentreueinformation anhand der VKBD und der KTBD,
    g) Ermitteln zumindest einer Annahmetreueinformation anhand der VMD und einer Annahme hinsichtlich einer Eigenschaft der VMD,
    h) Ermitteln einer Optimierungsinformation anhand der Datentreueinformation und der zumindest einen Annahmetreueinformation,
    i) Erzeugen der OTF durch Optimieren der VTF anhand der Optimierungsinformation.

Beispielsweise können die durch die OTF bereitgestellten Magnetfelddaten dazu verwendet werden, um räumliche Amplitudenschwankungen und/oder Inhomogenitäten in Magnetresonanzabbildungen zu reduzieren. Vorteilhafterweise spiegeln sich solche räumliche Amplitudenschwankungen und/oder Inhomogenitäten in den Magnetfelddaten wider.

Die Schritte a), b) und c) können insbesondere mittels jeweils dafür geeigneten Bereitstellungseinheiten und/oder Schnittstellen durchgeführt werden. Die Schritte d), e), f), g), h), und i) können insbesondere mittels einer dafür geeigneten Berechnungseinheit durchgeführt werden. Eine solche Berechnungseinheit kann insbesondere einen oder mehrere Prozessoren und/oder Speichermodule umfassen.

Die Schritte a) bis i), insbesondere die Schritte a) bis c) und/oder die Schritte f) und g), müssen nicht notwendigerweise in dieser (alphabetischen) Reihenfolge durchgeführt werden.

Vorzugsweise bildet eine trainierte Funktion, insbesondere die VTF und die OTF, Eingabedaten auf Ausgabedaten ab. Hierbei können die Ausgabedaten insbesondere weiterhin von einem oder mehreren Parametern der trainierten Funktion abhängen. Die trainierte Funktion ist insbesondere eine mittels maschinellen Lernens trainierte Funktion. Andere Begriffe für trainierte Funktion können beispielsweise trainierte Abbildungsvorschrift, Abbildungsvorschrift mit trainierten Parametern, Funktion mit trainierten Parametern, Algorithmus basierend auf künstlicher Intelligenz oder Algorithmus des maschinellen Lernens sein. Ein Beispiel für eine trainierte Funktion ist ein künstliches neuronales Netzwerk, wobei Kantengewichte des künstlichen neuronalen Netzwerks den Parametern der trainierten Funktion entsprechen. Anstatt des Begriffs "neuronales Netzwerk" kann auch der Begriff "neuronales Netz" verwendet werden. Insbesondere kann eine trainierte Funktion auch ein tiefes künstliches neuronales Netzwerk sein (ein englischer Fachbegriff ist "deep neural network" oder "deep artificial neural network"). Ein weiteres Beispiel für eine trainierte Funktion ist eine "Support Vector Machine", weiterhin sind auch insbesondere andere Algorithmen des maschinellen Lernens als trainierte Funktion einsetzbar.

Die Magnetfelddaten, insbesondere die VMD, können insbesondere Daten sein, die eine oder mehrere Eigenschaften eines Magnetfeldes beschreiben. Vorzugsweise handelt es sich dabei um ein Magnetfeld in einem Untersuchungsbereich einer Magnetresonanzvorrichtung. In dem Untersuchungsbereich kann während einer Magnetresonanzmessung mittels der Magnetresonanzvorrichtung ein zu untersuchendes Untersuchungsobjekt angeordnet werden. Das Untersuchungsobjekt kann beispielsweise sein Patient sein.

Die TBD können insbesondere Messdaten umfassen, die mit einer Magnetresonanzvorrichtung erfasst wurden, wie beispielsweise Rohdaten, insbesondere k-Raumdaten, und/oder Daten, die aus solchen Messdaten abgeleitet, insbesondere berechnet, wurden, wie beispielsweise eine oder mehrere aus Rohdaten und/oder k-Raumdaten rekonstruierte Magnetresonanzabbildungen. Insbesondere können diese Messdaten bei einer Magnetresonanzuntersuchung eines Untersuchungsobjekts erfasst worden sein. Insbesondere können die TBD Informationen über eine Anatomie eines Untersuchungsobjekts umfassen.

Die TBD können insbesondere ein kombiniertes Magnitudenbild umfassen. Es können insbesondere durch mehrere Spulenelemente einer Empfangsspulenanordnung der Magnetresonanzvorrichtung jeweils Magnetresonanzsignale empfangen werden, anhand derer ein kombiniertes Magnitudenbild erstellt werden können, d.h. die jeweiligen Signale werden kombiniert. Vorzugsweise umfassen die TBD Messdaten aus verschiedenen Magnetresonanzmessungen, insbesondere von verschiedenen Untersuchungsobjekten und/oder Teilen, insbesondere Körperteilen, der Untersuchungsobjekte. Beispielsweise wird die VTF nacheinander mit verschiedenen Rohdaten und/oder Magnetresonanzabbildungen trainiert.

Die KTBD können insbesondere hinsichtlich einer Eigenschaft eines Magnetfeldes, beispielsweise einer Inhomogenität eines Magnetfeldes, korrigiert sein. Zur Korrektur der TBD und einer daraus resultierenden Bereitstellung der KTBD können zahlreiche konventionelle, insbesondere bildbasierte, Korrekturverfahren eingesetzt werden, wie beispielsweise Statistical Parametric Mapping (SPM), Nonparametric Nonuniform intensity Normalization (N3), N4ITK, Uniform Combined Reconstruction (UNICORN) und/oder InhomoNet. Da das Training der Funktion, also das Erzeugen der OTF, keiner besonderen Zeitbeschränkung unterliegt, können solche Korrekturverfahren auch problemlos rechenaufwändig und zeitintensiv sein. Vorteilhafterweise ist jedoch das Resultat des Trainings, also die OTF, ausgebildet, in kurzer Zeit, insbesondere im Rahmen eines üblichen Magnetresonanzmessung, Magnetfelddaten bereitzustellen.

Vorteilhafterweise kann die OTF gemäß dem vorgeschlagenen Verfahren erzeugt bzw. trainiert werden, um mehrere Anatomien abzudecken. Konventionelle Verfahren sind hierfür oftmals weniger geeignet. So funktioniert beispielsweise der SPM-Algorithmus typischerweise nur gut für das Gehirn , und für den N4-Algorithmus müssen normalerweise unterschiedliche Parametersätze pro Anatomie bereitgestellt werden.

Die VTF kann insbesondere auf einem neuronalen Netzwerk mit vorläufigen, noch nicht optimierten, Kantengewichten als Parameter der VTF basieren. Beispielsweise werden die Kantengewichte zunächst gleich 1 gesetzt, und es wird als Architektur insbesondere ein UNET verwendet. Die erzeugte OTF kann insbesondere eine optimierte VTF sein. Beispielsweise können im Rahmen des Optimierens der VTF in Schritt i) vorläufige Kantengewichte des neuronalen Netzwerks geändert werden.

Vorzugsweise werden die TBD als Eingabedaten der VTF empfangen und auf die VTF angewendet. Vorzugsweise werden die VMD als Ausgabedaten der VTF ausgegeben.

Das Erzeugen der VKBD umfasst insbesondere eine Korrektur der TBD hinsichtlich einer Eigenschaft eines Magnetfeldes, beispielsweise einer Inhomogenität eines Magnetfeldes anhand der VMD.

Das Ermitteln der Datentreueinformation umfasst beispielsweise ein Anwenden einer Verlustfunktion (engl. loss function) und/oder Kostenfunktion (engl. cost function) auf die VKBD und die KTBD. Vorzugsweise beschreibt die Dateninformation eine Ähnlichkeit und/oder Datenkonsistenz und/oder Datentreue zwischen den VKBD und den KTBD. Beispielsweise kann die Datentreueinformation als ein Datentreuewert ausgedrückt werden. Insbesondere ist der Betrag des Datentreuewertes umso kleiner ist, je größer die Ähnlichkeit und/oder Datenkonsistenz und/oder Datentreue zwischen den VKBD und den KTBD ist. Wären beispielsweise VKBD und KTBD identisch, könnte der Datentreuewert Null betragen. Vorteilhafterweise wird mit Hilfe der Datentreueinformation eine (möglichst weitgehende) Datentreue und/oder Datenkonsistenz von mittels den Ausgabedaten der trainierten Funktion, also den Magnetfelddaten, korrigierte Bilddaten mit auf andere Weise korrigierten Bilddaten sichergestellt.

Es können insbesondere eine oder mehrere Annahmetreueinformationen ermittelt werden, denen jeweils eine andere Annahme zugrunde liegt. Jeder dieser Annahmen kann unterschiedliche Eigenschaften der VMD betreffen. Die zumindest eine Annahmetreueinformation kann für jede Annahme zumindest einen Annahmetreuewert umfassen. Vorzugsweise wird die OTF derart durch das Verfahren optimiert, dass die durch die OTF ausgegebenen Magnetfelddaten die Annahme (möglichst weitgehend) erfüllt. Die zumindest eine Annahme ist vorzugsweise zumindest eine zu erfüllende Annahme, insbesondere eine Bedingung.

Die Eigenschaft der VMD kann insbesondere eine Ausprägung, eine Eigenart und/oder eine Charakteristik und/oder Struktur der VMD sein. Diese Annahme kann insbesondere eine allgemeine und/oder grundsätzliche und/oder nicht-spezifische Annahme sein. Insbesondere hängt die Annahme nicht von den konkreten TBD ab, die als Eingabedaten der VTF bereitgestellt wurden. Die Annahme kann insbesondere eine Annahme über zumindest eine allgemeine und/oder grundsätzliche und/oder nicht-spezifische Eigenschaft von in Magnetresonanzvorrichtungen üblicherweise auftretenden Magnetfeldern umfassen. Die Eigenschaft kann insbesondere eine Ausprägung und/oder eine Eigenart und/oder eine Charakteristik und/oder Struktur solcher Magnetfelder betreffen. Die Annahme kann insbesondere auf einem Vorwissen über solche Magnetfelder beruhen.

Vorzugsweise werden zum Optimieren der VTF die Schritte c) bis i) iterativ, insbesondere wiederholt, durchgeführt, wobei die in Schritt i) erzeugte OTF als VTF in Schritt c) für eine nachfolgende Iteration, insbesondere Wiederholung, bereitgestellt wird. Das Verfahren kann einen oder mehrere solcher Iterationen umfassen.

Beispielsweise umfasst das Verfahren ein iteratives Minimieren zumindest eines Optimierungswertes der Optimierungsinformation. Idealerweise wird dann der Optimierungswert von Iteration zu Iteration tendenziell immer kleiner.

Vorzugsweise umfasst eine Iteration eine Auswertung der in Schritt h) ermitteln Optimierungsinformation und/oder der in Schritt g) ermittelten Annahmetreueinformation und/oder der in Schritt f) ermittelten Datentreueinformation, um in einer nachfolgenden Iteration die VTF gezielt zu optimieren und die OTF zu erzeugen. Insbesondere erfolgt ein Vergleich der in Schritt h) ermitteln Optimierungsinformation und/oder der in Schritt g) ermittelten Annahmetreueinformation und/oder der in Schritt f) ermittelten Datentreueinformation mit einer in Schritt h) ermitteln Optimierungsinformation und/oder einer in Schritt g) ermittelten Annahmetreueinformation und/oder einer in Schritt f) ermittelten Datentreueinformation einer vorangehenden Iteration, um in einer nachfolgenden Iteration die VTF gezielt zu optimieren.

Ist beispielsweise ein in Schritt h) ermittelter Optimierungswert der Optimierungsinformation in Iteration N-1 größer als in Iteration N, und ist ein möglichst kleiner Optimierungswert anzustreben, dann wird in Iteration N+1 vorteilhafterweise eine Optimierung der VTF gegenüber der Iteration N in einer gleichgerichteten Weise durchgeführt wie bei der Iteration N gegenüber der Iteration von N-1. Ist dagegen beispielsweise ein in Schritt h) ermittelter Optimierungswert der Optimierungsinformation in Iteration N-1 kleiner als in Iteration N, und ist ein möglichst kleiner Optimierungswert anzustreben, dann wird in Iteration N+1 vorteilhafterweise eine Optimierung der VTF gegenüber der Iteration N in einer entgegengerichteten Weise durchgeführt wie bei der Iteration N gegenüber der Iteration von N-1.

Vorzugsweise werden die Schritte d) bis i) so oft iterativ, insbesondere wiederholt, durchgeführt, bis die Optimierungsinformation eine vorgegebene Bedingung erfüllt. Eine solche vorgegebene Bedingung kann als Abbruchbedingung aufgefasst werden.

Beispielsweise umfasst die Optimierungsinformation einen Optimierungswert, und die Schritte d) bis i) werden so oft iterativ, insbesondere wiederholt, durchgeführt, bis der Optimierungswertes einen vorgegebenen Grenzwert unterschreitet.

Der Grenzwert ist vorzugsweise so gewählt bzw. vorgegeben, dass die VMD bei Unterschreitung dieses Grenzwertes eine hinreichend gute Qualität aufweisen bzw. die VTF so weit optimiert ist, eine VMD mit hinreichend guter Qualität ausgeben zu können.

Vorzugsweise beschreiben die VMD ein B1-Feld, insbesondere eine B1-Karte, und/oder ein B0-Feld, insbesondere eine B0-Karte, und/oder ein Verzerrungsfeld (engl. bias field), insbesondere eine Verzerrungskarte. Insbesondere können die VMD eine B1-Karte und/oder eine B0-Karte und/oder eine Verzerrungskarte sein. Das Verzerrungsfeld kann insbesondere ein Feld sein, das Intensitätsstörungen und/oder Intensitätsvariationen aufgrund von B1-Inhomogenitäten und/oder B0-Inhomogenitäten beschreibt.

Eine B1-Karte kann insbesondere als eine Repräsentation einer zweidimensionalen oder dreidimensionalen räumlichen Verteilung eines B1-Feldes angesehen werden. Eine B0-Karte kann insbesondere als eine Repräsentation einer zweidimensionalen oder dreidimensionalen räumlichen Verteilung eines B0-Feldes angesehen werden. Eine Verzerrungskarte kann insbesondere als eine Repräsentation einer zweidimensionalen oder dreidimensionalen räumlichen Verteilung eines Verzerrungsfeldes angesehen werden.

Vorzugsweise beschreiben die VMD eine B1-Karte und/oder Verzerrungskarte, und die dem Schritt g) zugrundliegende Annahme betrifft eine Weichheit (engl. smoothness) dieser B1-Karte bzw. Verzerrungskarte. Vorzugsweise wird angenommen, dass die B1-Karte bzw. die Verzerrungskarte möglichst weich ist, insbesondere möglichst keine bzw. möglichst schwache Kanten aufweist und/oder eine geringe räumliche Auflösung aufweist. Beispielsweise kann empirisch ein Maß für die räumliche Auflösung bestimmt werden, die als (Ziel-)Annahme verwendet werden kann. Vorteilhafterweise wird durch diese Annahme die OTF trainiert, etwaige in den Eingabedaten, also den Bilddaten, enthaltene Anatomieinformationen in den Ausgabedaten, also den Magnetfelddaten, zu unterdrücken.

Vorzugsweise umfasst das Ermitteln der Optimierungsinformation eine relative Gewichtung der Datentreueinformation und der zumindest einen Annahmetreueinformation. Die Gewichtung, insbesondere etwaige die Gewichtung beschreibende Gewichtungsfaktoren, kann beispielsweise empirisch bestimmt werden.

Insbesondere kann die Optimierungsinformation einen Optimierungswert umfassen, der eine gewichtete Summe des Datentreuewertes und des zumindest einen Annahmetreuewertes ist, wobei die Koeffizienten der Summe Gewichtungsfaktoren darstellen, z.B. $OW=DTW+\sum_n c_n \cdot ATW_n$, wobei OW der Optimierungswert, DTW der Datentreuewert, $c_n$ die Gewichtungsfaktoren und $ATW_n$ die Annahmetreuewerte sind.

Die iterative Optimierung der VTF zum Erzeugen der OTF kann dann beispielsweise als Optimierungsproblem min(OW), d.h. minimiere den Optimierungswert, ausgedrückt werden, das durch eine iterative Anpassung der VTF, insbesondere ihrer Gewichte bzw. Kantengewichte, gelöst wird.

Vorzugsweise sind die TBD und/oder die KTBD komprimierte, insbesondere downgesampelte, Bilddaten. Somit kann die mit den Bilddaten verknüpfte Datenmenge reduziert werden, was wiederum einen Berechnungsaufwand zum Erzeugen der OTF verringern kann. Die Kompression der Bilddaten kann insbesondere eine Reduzierung einer räumlichen Auflösung der Bilddaten umfassen.

Beispielsweise werden ursprüngliche Trainings-Bilddaten, $TBD_i$, erfasst. Aus diesen werden mittels einer Kompression die TBD erzeugt. Ferner können anhand der $TBD_i$ ursprüngliche korrigierte Trainings-Bilddaten, $KTBD_i$, erzeugt werden, anhand derer dann wiederum durch Kompression die KTBD erzeugt werden können.

Vorzugsweise liegt dem VTF ein neuronales Netz, insbesondere ein Convolutional Neural Network (CNN), insbesondere ein U-Net, zugrunde.

Ein U-Net umfasst vorzugsweise ein Netz mit einem Kontraktionspfad und einem Expansionspfad. Im Kontraktionspfad wird üblicherweise eine räumliche Information reduziert, während eine Merkmalsinformation erhöht wird, und im Expansionspfad werden Merkmalsinformation und die räumlichen Informationen wieder kombiniert. Vorteilhafterweise kann dadurch eine Auflösung der Ausgabedaten erhöht werden.

Vorzugsweise werden zum Optimieren der VTF, insbesondere zur Erzeugung der OTF, Gewichte, insbesondere Kantengewichte, des neuronalen Netzes, das der VTF zugrunde liegt, geändert.

Vorzugsweise umfasst das Erzeugen der VKBD eine Multiplikation einer B1-Karte, die durch die VMD beschrieben wird, mit einer Magnetresonanzabbildung, die durch die TBD beschrieben wird. Vorteilhafterweise können durch diese Multiplikation etwaige durch Inhomogenitäten des B1-Feldes (die durch die B1-Karte beschrieben werden) verursachte Bildartefakte in der Magnetresonanzabbildung korrigiert werden.

Vorzugsweise umfasst das Ermitteln der Datentreueinformation eine Anwendung einer Kostenfunktion, insbesondere einer $L_1$ - Kostenfunktion, auf die VKBD und die KTBD. Insbesondere umfasst das Ermitteln der Datentreueinformation ein Berechnen eines Vergleichs-wertes, insbesondere eines $L_1$-Verlusts, zwischen den VKBD und der KTBD umfasst. Der DTW kann beispielsweise folgendermaßen, insbesondere als Strafterm des Optimierungsverfahrens zur Erzeugung der OTF, dargestellt werden:

$$DTW=\|VKBD-KTBD\|_1$$

Vorzugsweise umfasst das Ermitteln der zumindest einen Annahmetreueinformation eine Erzeugung von k-Raumdaten, kRD, anhand einer B1-Karte und/oder Verzerrungskarte, die durch die VMD beschrieben wird, wobei die Erzeugung der kRD eine Fouriertransformation der B1-Karte und/oder der Verzerrungskarte in einen k-Raum umfasst, wobei Vergleichs-k-Raumdaten, VkRD, anhand der kRD erzeugt werden, wobei das Ermitteln der zumindest einen Annahmetreueinformation eine Anwendung einer Kostenfunktion, insbesondere einer L1-Kostenfunktion, auf die kRD und die Vergleichs-k-Raumdaten, umfasst. Der ATW kann beispielsweise folgendermaßen, insbesondere als weiterer Strafterm des Optimierungsverfahrens zur Erzeugung der OTF, dargestellt werden:

$$ATW=\|kRD-VkRD\|_1$$

Durch die Fouriertransformation wird die B1-Karte vorteilhafterweise in den k-Raum transformiert. Der k-Raum kann insbesondere ein Ortsfrequenzraum sein. Der k-Raum kann insbesondere mit einem zweidimensionalen Datenmodell beschrieben werden.

Die Fouriertransformation kann insbesondere eine zweidimensionale Fouriertransformation sein. Die Fouriertransformation kann insbesondere eine schnelle Fourier-Transformation (engl. Fast Fourier Transform, FFT) sein.

Vorzugsweise umfasst das Erzeugen der VkRD ein Nullsetzen von Werten der kRD in zumindest einem Segment des k-Raums, das außerhalb eines vorgegebenen, insbesondere zentralen, Segments der k-Raums liegt.

Vorteilhafterweise eignen sich ein solchermaßen erzeugte VkRD, um mittels Anwendung der Kostenfunktion, insbesondere der L1-Kostenfunktion, als Annahmetreuewert ATW ein Maß für eine Weichheit der B1-Karte bzw. Verzerrungskarte zu ermitteln. Insbesondere können dadurch die VTF dahingehend trainiert werden, dass es etwaige störende Informationen über eine Anatomie eines Untersuchungsobjekts, das zur Bereitstellung der TBD gemessen wurde, bei der Erzeugung der VMD unterdrückt werden.

Beispielsweise umfasst der k-Raum 128x128 Punkte, wobei das vorgegebene Segment 32x32 Punkte umfasst, die im Zentrum des k-Raums liegen, und die Werte der k-Raumpunkte, die außerhalb dieses zentralen Segments liegen, Null gesetzt werden.

Ferner wird ein computerimplementiertes Verfahren zum Bereitstellen von Magnetfelddaten, insbesondere einer B1-Karte und/oder eine B0-Karte und/oder Verzerrungskarte, mittels einer trainierten Funktion vorgeschlagen. Dieses Verfahren umfasst:

- Empfangen von Bilddaten als Eingabedaten der trainierten Funktion,
- Anwenden der trainierten Funktion auf die Bilddaten, wobei die trainierte Funktion basierend auf

   i) einer Datentreue von anhand der Magnetfelddaten korrigierten Bilddaten
   und
   ii) zumindest einer Annahme über zumindest eine Eigenschaft der Magnetfelddaten
   trainiert ist,

- Bereitstellung der Magnetfelddaten als Ausgabedaten der trainierten Funktion.

Vorteilhafterweise kann die trainierte Funktion die Magnetfelddaten schnell und zuverlässig bereitstellen. Vorteilhafterweise beträgt eine typische Berechnungszeit nur wenige Sekunden für ein ganzes (dreidimensionales) 3D-Volumen, während konventionelle Algorithmen typischerweise mehrere Minuten benötigen.

Vorzugsweise ist die trainierte Funktion eine optimierte trainierte Funktion, OTF, die mit einem Verfahren vorab beschriebenen Verfahren zum Erzeugen der OTF erzeugt wurde. Etwaige Vorteile dieses Verfahrens können auf das Verfahren zur Anwendung der trainierten Funktion übertragen werden.

Das Empfangen der Bilddaten kann insbesondere mittels einer dafür geeigneten Empfangseinheit und/oder Schnittstelle durchgeführt werden. Das Anwenden der OTF auf die Eingabedaten kann insbesondere mittels einer dafür geeigneten Berechnungseinheit durchgeführt werden. Eine solche Berechnungseinheit kann insbesondere einen oder mehrere Prozessoren und/oder Speichermodule umfassen. Eine solche Berechnungseinheit kann insbesondere ein Teil einer Systemsteuereinheit einer Magnetresonanzvorrichtung sein.

Vorteilhafterweise benötigt die trainierte Funktion als Eingabedaten lediglich die Bilddaten, so dass auf etwaige zusätzliche Magnetresonanzmessungen, insbesondere Vormessungen und/oder Justagemessungen, verzichtet werden kann. Vorteilhafterweise ist Anwendung der OTF entkoppelt von einer Rekonstruktion von Magnetresonanzabbildungen.

Vorteilhafterweise werden anhand der bereitgestellten Magnetfelddaten Steuerdaten zur Durchführung einer Magnetresonanzmessung ermittelt werden. Vorzugsweise wird mittels der ermittelten Steuerdaten eine Magnetresonanzmessung durchgeführt.

Die Steuerdaten können beispielsweise geeignet sein, um mittels einer Magnetresonanzvorrichtung pTx-Pulse auszugeben. Insbesondere umfassen die Steuerdaten für mehrere Sendespulen einer Sendespulenanordnung jeweils eine Form und/oder eine Amplitude und/oder Phase eines Teilpulses und/oder eine zeitliche Verzögerung zwischen den Teilpulsen und/oder eine Anzahl an Teilpulsen. Beispielsweise setzt sich ein emittierbarer HF-Sendepuls aus mehreren Teilpulsen zusammen, die voneinander abweichen und jeweils durch eine Sendespule einer mehrkanaligen Sendespulenanordnung gesendet werden können, d.h. es handelt sich bei dem emittierbaren HF-Sendepuls um einen pTx-Puls. Ein pTx-Puls kann ferner auch zumindest einen Gradientenpuls umfassen. Diese Gesamtheit der Teilpulse eines solchen Mehrkanal-Pulses bzw. des zumindest einen Gradientenpulses kann insbesondere durch die Steuerdaten beschrieben werden.

Durch einen pTx-Puls kann vorteilhafterweise das dadurch erzeugte B1-Feld präziser kontrolliert werden; eine solche Kontrolle kann insbesondere vorteilhaft sein bei Anwendungen mit reduziertem Sichtfeld, geformten Sättigungsbändern oder zur Reduktion der spezifischen Absorptionsrate (engl. specific absorption rate, SAR). Insbesondere können mit einem pTx-Puls Magnetfeldinhomogenitäten ausgeglichen werden (beispielsweise im Rahmen eines "HF-Shimming"), das vor allem bei höheren Feldstärken des Hauptmagnetfelds ab 7 Tesla besonders vorteilhaft sein kann.

Die zumindest eine Form und/oder Amplitude und/oder Phase des HF-Sendepulses bzw. eines Teilpulses kann beispielsweise einer Form und/oder Amplitude und/oder Phase eines Spannungspulses, der an die Sendespulenanordnung angelegt wird, und/oder eines Strompulses, der durch die Sendespulenanordnung fließt, entsprechen.

Die zumindest eine Form und/oder Amplitude und/oder Dauer des Gradientenpulses kann beispielsweise einer Form und/oder Amplitude und/oder Dauer eines Spannungspulses, der an die Gradientenspuleneinheit angelegt wird, und/oder eines Strompulses, der durch die Gradientenspuleneinheit fließt, entsprechen.

Insbesondere Inhomogenitäten des B1-Feldes können zu störenden Signal- und Kontrastvariationen führen. Vorteilhafterweise werden diese Variationen noch während der Magnetresonanzmessung korrigiert. Vorteilhafterweise werden dazu geeignete pTx-Pulse verwendet, die mit Hilfe der durch die OTF ermittelten Magnetfelddaten ermittelt werden.

Ferner wird eine Systemsteuereinheit vorgeschlagen, die ausgebildet ist, ein vorangehend beschriebenes Verfahren zum Bereitstellen von Magnetfelddaten durchzuführen. Ferner wird eine Magnetresonanzvorrichtung mit einer solchen Systemsteuereinheit vorgeschlagen.

Die Vorteile der vorgeschlagenen Systemsteuereinheit und der Magnetresonanzvorrichtung entsprechen im Wesentlichen den Vorteilen des vorgeschlagenen Verfahrens zum Bereitstellen von Magnetfelddaten, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Ferner wird ein Computerprogrammprodukt vorgeschlagen, das ein Programm umfasst und direkt in einen Speicher einer (programmierbaren) Systemsteuereinheit einer Magnetresonanzvorrichtung ladbar ist und Programmmittel, z.B. Bibliotheken und Hilfsfunktionen, aufweist, um ein vorgeschlagenes Verfahren auszuführen, wenn das Computerprogrammprodukt in der Systemsteuereinheit der Magnetresonanzvorrichtung ausgeführt wird. Das Computerprogrammprodukt kann dabei eine Software mit einen Quellcode, der noch kompiliert und gebunden oder der nur interpretiert werden muss, oder einen ausführbaren Softwarecode umfassen, der zur Ausführung nur noch in die Systemsteuereinheit zu laden ist.

Durch das Computerprogrammprodukt kann das vorgeschlagene Verfahren schnell, identisch wiederholbar und robust ausgeführt werden. Das Computerprogrammprodukt ist so konfiguriert, dass es mittels der Systemsteuereinheit die vorgeschlagene Verfahrensschritte ausführen kann. Die Systemsteuereinheit weist vorteilhafterweise dabei jeweils die Voraussetzungen wie beispielsweise einen entsprechenden Arbeitsspeicher, eine entsprechende Grafikkarte oder eine entsprechende Logikeinheit auf, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Systemsteuereinheit geladen werden kann, der mit der Magnetresonanzvorrichtung direkt verbunden oder als Teil der Magnetresonanzvorrichtung ausgebildet sein kann.

Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Systemsteuereinheit einer Magnetresonanzvorrichtung ein vorgeschlagenes Verfahren durchführen. Beispiele für elektronische lesbare Datenträger sind eine DVD, ein Magnetband oder einen USB-Stick, auf welchem elektronisch lesbare Steuerinformationen, insbesondere Software, gespeichert ist. Wenn diese Steuerinformationen von dem Datenträger gelesen und in eine Systemsteuereinheit der Magnetresonanzvorrichtung gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:

Fig. 1     eine Magnetresonanzvorrichtung in einer schematischen Darstellung,

Fig. 2     ein Verfahren zum Erzeugen einer optimierten trainierten Funktion zur Bereitstellung von Magnetfeld-daten in einer schematischen Darstellung,

Fig. 3     verschiedene mögliche Aspekte eines Verfahrens zum Erzeugen einer optimierten trainierten Funktion,

Fig. 4     Verfahren zum Bereitstellen von Magnetfelddaten mittels einer trainierten Funktion in einer schematischen Darstellung.

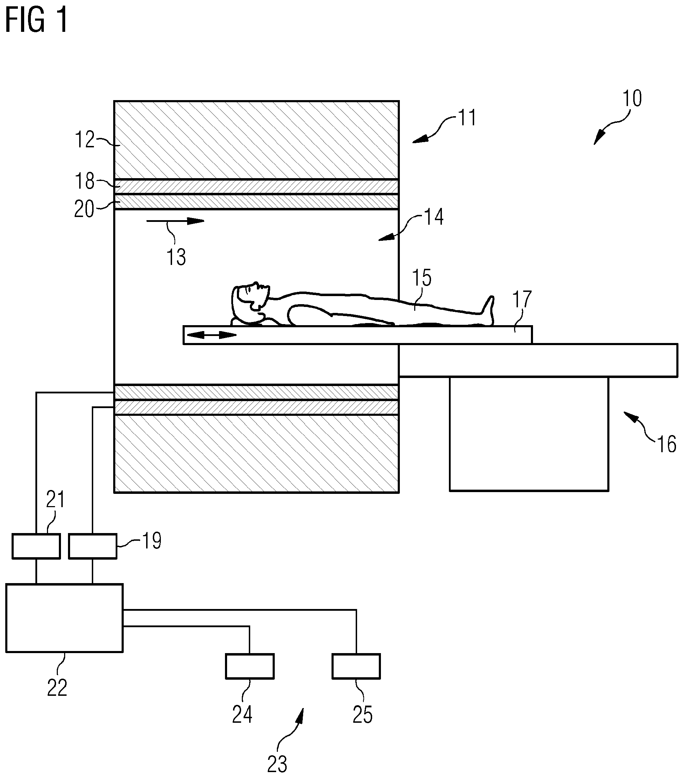

In Fig. 1 ist eine Magnetresonanzvorrichtung 10 schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds 13 aufweist. Das Hauptmagnetfeld 13 kann auch B0-Feld bezeichnet werden. Zudem umfasst die Magnetresonanzvorrichtung 10 einen Patientenaufnahmebereich 14 zu einer Aufnahme eines Patienten 15. In der Mitte des Patientenaufnahmebereich 14, in dem das Hauptmagnetfeld 13 eine besonders hohe Homogenität aufweist, befindet sich der Untersuchungsbereich der Magnetresonanzvorrichtung 10. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert.

Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 wird von einer Hochfrequenzantennensteuereinheit 21 der Magnetresonanzvorrichtung 10 gesteuert. Die Hochfrequenzantenneneinheit 20 umfasst eine Sendespulenanordnung und eine Empfangsspulenanordnung, die in diesem Fall identisch ist, d.h. die Spulen, mit denen HF-Sendepulse emittiert werden, sind auch die Spulen, mit denen Magnetresonanzsignale empfangen werden. Das Sendefeld wird üblicherweise als B1$^+$-Feld bezeichnet und das Empfangsfeld als B1$^-$-Feld. Die HF-Sendepulse werden in den Untersuchungsbereich eingestrahlt. Dadurch stellt sich dem von dem Hauptmagneten 12 erzeugten Hauptmagnetfeld 13 eine Anregung von Atomkernen ein. Durch Relaxation der angeregten Atomkerne werden Magnetresonanzsignale erzeugt. Die Hochfrequenzantenneneinheit 20 ist zum Empfang der Magnetresonanzsignale ausgebildet.

Gerade in Magnetresonanzvorrichtungen mit hohen Stärken des Hauptmagnetfeldes 13, von z.B. 7 Tesla, sind die Sende- und Empfangsspulenanordnungen üblicherweise nicht Teil einer fest in die Magnetresonanzvorrichtung 10 integrierten Körperspulen, sondern es werden - hier nicht abgebildete - lokale Sende- und Empfangsspulenanordnungen unmittelbar an den Patienten 15 angeordnet. Solche Sende- und Empfangsspulenanordnungen umfassen insbesondere mehrere Sende- und/oder Empfangskanäle, so dass sie für ein paralleles Senden und/oder Empfangen geeignet sind.

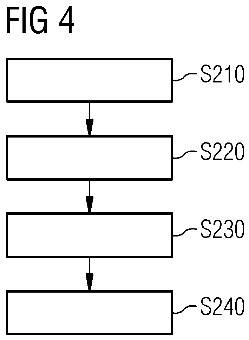

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Die Systemsteuereinheit 22 ist vorzugsweise ausgebildet, ein Verfahren zum Bereitstellen von Magnetfelddaten mittels einer trainierten Funktion gemäß Fig. 4 durchzuführen. Zudem umfasst die Systemsteuereinheit 22 eine nicht näher dargestellte Auswerteeinheit zu einer Auswertung der Magnetresonanzsignale, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzabbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

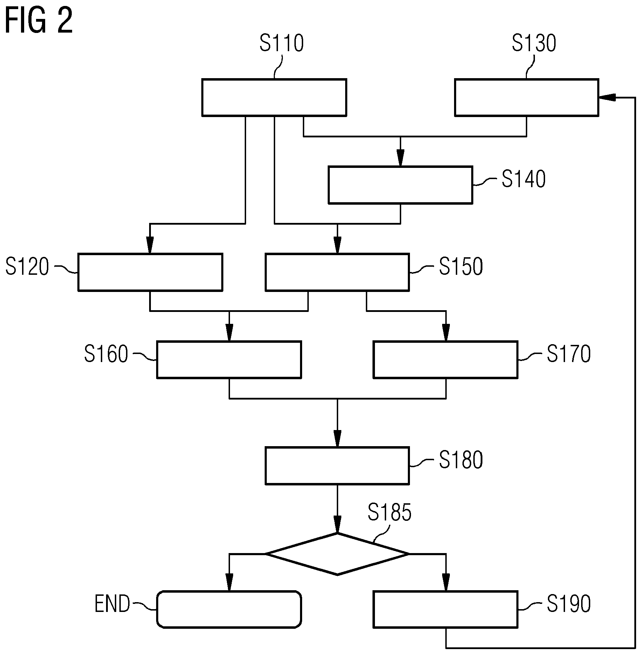

In Fig. 2 ist ein computerimplementiertes Verfahren zum Erzeugen einer optimierten trainierten Funktion, OTF, zur Bereitstellung von Magnetfelddaten schematisch dargestellt. In S110 werden Trainings-Bilddaten, TBD, bereitgestellt. In S120 werden anhand der TDB erzeugte korrigierte Trainings-Bilddaten, KTBD, bereitgestellt. In S130 wird eine vorläufige trainierte Funktion, VTF, bereitgestellt. In S140 werden vorläufiger Magnetfelddaten, VMD, durch Anwenden der TBD auf die VTF erzeugt. Die VMD können beispielsweise ein B1-Feld, insbesondere eine B1-Karte, und/oder ein B0-Feld, insbesondere eine B0-Karte, und/oder ein Verzerrungsfeld beschreiben. In S150 werden vorläufige korrigierte Bilddaten, VKBD, anhand der VMD und der TBD erzeugt. In S160 werden Datentreueinformation anhand der VKBD und der KTBD ermittelt. In S170 wird zumindest eine Annahmetreueinformation anhand der VKBD und einer Annahme hinsichtlich der VMD ermittelt. In S180 wird anhand der Datentreueinformation und der zumindest einen Annahmetreueinformation eine Optimierungsinformation ermittelt. In S185 wird überprüft, ob die Optimierungsinformation eine vorgegebene Bedingung erfüllt. Falls die vorgegebene Bedingung erfüllt ist, wird das Verfahren in END beendet, d.h. die Optimierung der VTF ist dann abgeschlossen. Die VTF ist dann die OTF. Falls die vorgegebene Bedingung (noch) nicht erfüllt ist, wird die Optimierung fortgeführt, indem S190 die VTK geändert wird. Die in S190 geänderte VTK ist dann die (neue) VTK in S130. In einer weiteren Iteration werden die Schritte S130 bis S180 nochmals durchgeführt, um in S185 wiederum zu überprüfen, ob die vorgegebene Bedingung erfüllt ist. Es werden also gegebenenfalls mehrere Iterationen durchgeführt, um die OTF zu erzeugen.

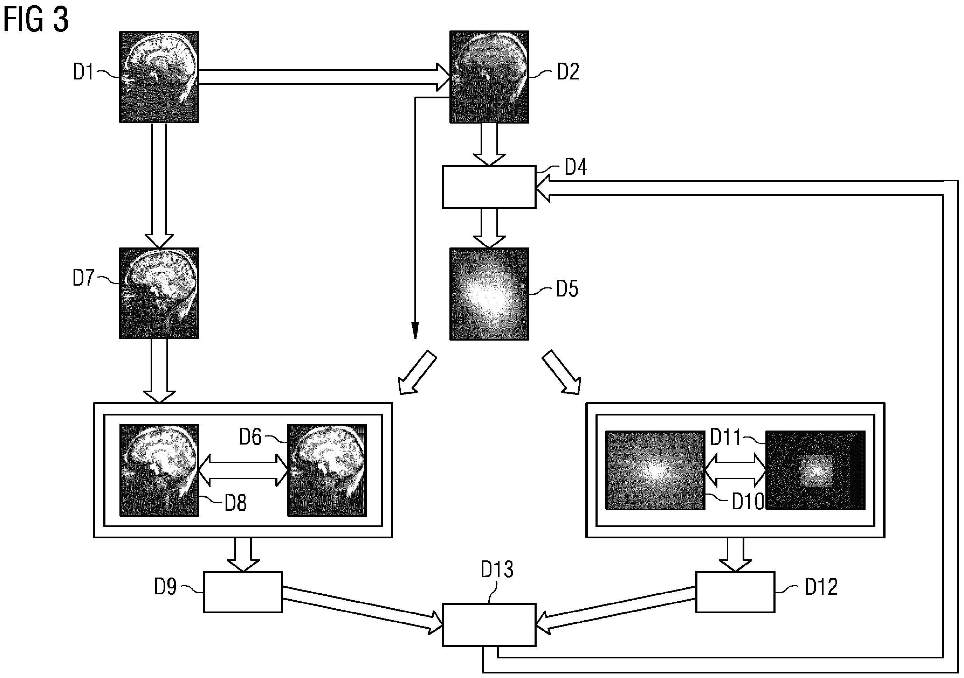

Anhand von Fig. 3 sollen verschiedene Aspekte eines möglichen Ausführungsbeispiels genauer erläutert werden. Es werden ursprünglichen Trainings-Bilddaten, TBD$_i$, D1 bereitgestellt. Diese TBD$_i$ D1 können beispielsweise durch eine Magnetresonanzmessung mittels der Magnetresonanzvorrichtung 10 aufgenommen werden oder aus mittels der Magnetresonanzvorrichtung 10 aufgenommen Daten abgeleitet, insbesondere berechnet, werden.

Aus den TBD$_i$ D1 wird durch eine Kompression, genauer: durch eine Reduktion der Auflösung, Trainings-Bilddaten, TBD, D2 erzeugt. Hier wird die Auflösung auf 128x128 Bildpunkte herabgesetzt (engl. downsampling). Die Kompression der TBD$_i$ D1 kann eine im weiteren Verlauf (insbesondere in S140, S150, S160, S170 und S180) notwendige Rechenlast reduzieren, da eine geringere Datenmenge verarbeitet werden muss; die Kompression ist aber nur optional.

Die TBD D2 werden gemäß S110 bereitgestellt und dienen als Eingabedaten für eine VTF D4, die gemäß S130 bereitgestellt wird. Die VTF D4 ist beispielsweise ein Deep-Learning-Netzwerk, dass auf U-Net basiert. Durch Anwenden der VTF D4 auf die TBD werden VMD gemäß S140 erzeugt. Diese VMD sind hier eine Karte eines Verzerrungsfelds D5, das Inhomogenitäten des B1-Feldes beschreibt.

Gemäß S150 werden VKBD D6 anhand der der VMD und der TBD D2 erzeugt. Dazu wird die Karte eines Verzerrungsfelds D5 mit den TBD D2 multipliziert, so dass in dem VKBD D6, um die Inhomogenitäten des B1-Feldes zu kompensieren. Gemäß S160 wird anhand der VKBD D6 und der KTBD D8 eine Datentreueinformation D9 ermittelt. Um die KTBD D8 zu generieren, werden die $TBD_i$ D1 hinsichtlich der Inhomogenitäten des B1-Feldes zunächst korrigiert, beispielsweise mit einem Korrekturverfahren wie Statistical Parametric Mapping (SPM), Nonparametric Nonuniform intensity Normalization (N3), N4ITK und/oder Uniform Combined Reconstruction (UNICORN), so dass man ursprüngliche korrigierte Trainings-Bilddaten, $KTBD_i$, D7 erhält. Die $KTBD_i$ D7 werden dann - analog zu den $TBD_i$, d.h. hier auf eine Auflösung von 128x128 Bildpunkten - komprimiert, um die KTBD D8 zu erhalten. (Es ist auch denkbar, zuerst die Kompression und danach die Korrektur durchzuführen.) Auch die Kompression der $KTBD_i$ ist nur optional.

Die Datentreueinformation D9 wird hier ermittelt, indem eine Kostenfunktion auf die VKBD und die KTBD angewendet wird, wobei die dabei ermittelten Kosten (engl. cost, loss) die Datentreueinformation D9 sind. Insbesondere eignet sich dazu eine L1-Kostenfunktion. Der Wert der Kosten ist umso geringer, je mehr die VKBD D6 den KTBD D8 entsprechen. Die KTBD D8 fungieren also als Ground-Truth-Daten gegenüber den VKBD D6.

Andererseits wird gemäß S170 zumindest eine Annahmetreueinformation D12 anhand der VKBD und einer Annahme hinsichtlich der VMD ermittelt. Dazu wird eine FFT der Karte des Verzerrungsfeldes D5 durchgeführt, so dass man k-Raumdaten, kRD D10 erhält. Die Karte des Verzerrungsfeldes D5 wird also von einem Bildraum in den k-Raum transformiert.

Aus den kRD D10 werden wiederum Vergleichs-k-Raumdaten, VkRD, D11 abgeleitet. Dies erfolgt hier, indem alle k-Raum-Werte außerhalb eines zentralen Bereichs des k-Raums auf Null gesetzt werden. Der zentrale Bereich ist in diesem Fall 32x32 k-Raum-Werte groß. Die Annahmetreueinformation D12 sind die Kosten einer Kostenfunktion, die auf die kRD D10 und die VkRD D11 angewendet wird. Insbesondere eignet sich hierbei eine L1-Kostenfunktion. Es wird hier also ein hoher Informationsgehalt in der Peripherie des k-Raums der kRD D10 pönalisiert.

Die Peripherie eines k-Raums enthält üblicherweise Informationen über Kanten im zugehörigen Bildraum, d.h. hier: Kanten in der Karte des Verzerrungsfeldes D5. Es wird jedoch angenommen, dass das Verzerrungsfeldes D5 eher keine Kanten aufweisen soll; vielmehr sind Kanten typisch für anatomische Merkmale des Untersuchungsobjekts, die sich nicht im Verzerrungsfeldes D5 niederschlagen sollen. Daher ist die Weichheit, also die "Kantenfreiheit", eine geeignete Annahme für die Verzerrungskarte.

In diesem Beispiel wird nur eine Annahme über die Verzerrungskarte vorgenommen bzw. nur eine Annahmetreueinformation ermittelt. Es ist aber auch denkbar, dass mehrere Annahmetreueinformation ermittelt werden.

Gemäß S180 wird anhand der Datentreueinformation D9 und der Annahmetreueinformation D12 eine Optimierungsinformation D13 ermittelt. Die Optimierungsinformation D13 kann beispielsweise eine gewichtete Summe der Kosten der Datentreueinformation D9 und die Kosten der Annahmetreueinformation D12. Die zugehörigen Gewichtungsfaktoren können beispielsweise empirisch festgelegt werden.

Ziel der Optimierung kann insbesondere sein, die Optimierungsinformation D13 (bzw. einen die Optimierungsinformation D13 beschreibenden Optimierungswert) zu minimieren. Gemäß S185 kann nun beispielsweise überprüft werden, ob die Optimierungsinformation D13 (bzw. der die Optimierungsinformation D13 beschreibender Optimierungswert) nun bereits klein genug ist. Falls dem so wäre, kann die Optimierung abgeschlossen werden, so dass die zuletzt verwendete VKF bereits das Ergebnis der Optimierung, also die OTF ist.

Falls die die Optimierungsinformation D13 (bzw. der die Optimierungsinformation D13 beschreibender Optimierungswert) nun (noch) nicht klein genug ist, wird die VKF modifiziert und mit der modifizierten VFK werden eine oder mehrere weitere Iterationen durchgeführt.

In Fig. 4 ist ein computerimplementiertes Verfahren zum Bereitstellen von Magnetfelddaten schematisch dargestellt. In S210 werden Bilddaten empfangen. In S220 werden die empfangenen Bilddaten auf eine trainierte Funktion angewendet, die basierend auf einer Datentreue von anhand der Magnetfelddaten korrigierten Bilddaten, und zumindest einer Annahme über zumindest eine Eigenschaft der Magnetfelddaten trainiert ist. Vorzugsweise handelt es sich bei der trainierten Funktion um eine OTF nach einem Erzeugungsverfahren gemäß Fig. 2 und/oder 3. In S230 werden die Magnetfelddaten ausgegeben und bereitgestellt. In S240 werden Steuerdaten zur Durchführung einer Magnetresonanzmessung anhand der bereitgestellten Magnetfelddaten ermittelt.

In S240 werden mit Hilfe einer Verzerrungskarte (als Ausgabedaten der OTF) beispielsweise pTx-Pulse designt. Diese pTx-Pulse können beispielsweise für eine T1-gewichtete, eine T2-gewichtete und/oder für eine diffusionsgewichtete Magnetresonanzmessung verwendet werden. Insbesondere kann mit Hilfe der durch die OTF ausgegebenen Magnetfelddaten ein HF-Shimming durchgeführt werden.

Es wird abschließend noch einmal darauf hingewiesen, dass es sich bei den vorhergehend detailliert beschriebenen Verfahren sowie bei der dargestellten Systemsteuereinheit und Magnetresonanzvorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

**Patentansprüche**

1. Computerimplementiertes Verfahren zum Erzeugen einer optimierten trainierten Funktion, OTF, zur Bereitstellung von Magnetfelddaten, mit den Schritten:

   a) Bereitstellen von Trainings-Bilddaten, TBD, (S110)
   b) Bereitstellen anhand der TDB erzeugter korrigierter Trainings-Bilddaten, KTBD, (S120)
   c) Bereitstellen einer vorläufigen trainierten Funktion, VTF, (S130)
   d) Erzeugen vorläufiger Magnetfelddaten, VMD, durch Anwenden der TBD auf die VTF, (S140)
   e) Erzeugen von vorläufigen korrigierten Bilddaten, VKBD, anhand der VMD und der TBD, (S150)
   f) Ermitteln einer Datentreueinformation anhand der VKBD und der KTBD, (S160)
   g) Ermitteln zumindest einer Annahmetreueinformation anhand der VMD und einer Annahme über zumindest eine Eigenschaft der VMD, (S170)
   h) Ermitteln einer Optimierungsinformation anhand der Datentreueinformation und der zumindest einen Annahmetreueinformation, (S180)
   i) Erzeugen der OTF durch Optimieren der VTF anhand der Optimierungsinformation.

2. Verfahren nach Anspruch 1,
   wobei die Schritte c) bis i) iterativ durchgeführt werden, wobei die in Schritt i) erzeugte OTF als VTF in Schritt c) für eine nachfolgende Iteration bereitgestellt wird.

3. Verfahren nach Anspruch 2,
   wobei die Schritte d) bis i) so oft iterativ durchgeführt werden, bis die Optimierungsinformation eine vorgegebene Bedingung erfüllt.

4. Verfahren nach einem der vorangehenden Ansprüche,
   wobei die VMD ein B1-Feld, insbesondere eine B1-Karte, und/oder ein B0-Feld, insbesondere eine B0-Karte, und/oder ein Verzerrungsfeld beschreiben.

5. Verfahren nach einem der vorangehenden Ansprüche,
   wobei die dem Schritt g) zugrundliegende Annahme eine Weichheit einer B1-Karte und/oder Verzerrungskarte betrifft, die durch die VMD beschrieben werden.

6. Verfahren nach einem der vorangehenden Ansprüche,
   wobei das Ermitteln der Optimierungsinformation eine relative Gewichtung der Datentreueinformation und der zumindest einen Annahmetreueinformation umfasst.

7. Verfahren nach einem der vorangehenden Ansprüche,
   wobei die TBD und/oder die KTBD komprimierte Bilddaten sind.

8. Verfahren nach einem der vorangehenden Ansprüche,

   wobei dem VTF ein neuronales Netz, insbesondere ein Convolutional Neural Network, insbesondere auf ein U-Net, zugrunde liegt,
   wobei zum Optimieren der VTF Gewichte des neuronalen Netzes, das der VTF zugrunde liegt, geändert werden.

9. Verfahren nach einem der vorangehenden Ansprüche,
   wobei das Erzeugen der VKBD eine Multiplikation einer B1-Karte, die durch die VMD beschrieben wird, mit einer Magnetresonanzabbildung, die durch die TBD beschrieben wird, umfasst.

10. Verfahren nach einem der vorangehenden Ansprüche,
    wobei das Ermitteln der Datentreueinformation eine Anwendung einer Kostenfunktion, insbesondere einer L1-Kostenfunktion, auf die VKBD und die KTBD umfasst.

11. Verfahren nach einem der vorangehenden Ansprüche,

    wobei das Ermitteln der zumindest einen Annahmetreueinformation eine Erzeugung von k-Raumdaten, kRD, anhand einer B1-Karte und/oder einer Verzerrungskarte, die durch die VMD beschrieben wird, umfasst,
    wobei die Erzeugung der kRD eine Fouriertransformation der B1-Karte und/oder der Verzerrungskarte in einen k-Raum umfasst,
    wobei Vergleichs-k-Raumdaten, VkRD, anhand der kRD erzeugt werden,
    wobei das Ermitteln der zumindest einen Annahmetreueinformation eine Anwendung einer Kostenfunktion, insbesondere einer L1-Kostenfunktion, auf die kRD und die Vergleichs-k-Raumdaten, umfasst.

12. Verfahren nach Anspruch 11,

wobei das Erzeugen der VkRD ein Nullsetzen von Werten in zumindest einem Segment der kRD, das außerhalb eines vorgegebenen, insbesondere zentralen, Segments der kRD liegt, umfasst.

13. Computerimplementiertes Verfahren zum Bereitstellen von Magnetfelddaten mittels einer trainierten Funktion, umfassend

    - Empfangen von Bilddaten als Eingabedaten der trainierten Funktion,
    - Anwenden der trainierten Funktion auf die Bilddaten,
    wobei die trainierte Funktion basierend auf

      i) einer Datentreue von anhand der Magnetfelddaten korrigierten Bilddaten
      und
      ii) zumindest einer Annahme über zumindest eine Eigenschaft der Magnetfelddaten trainiert ist,

    - Bereitstellung der Magnetfelddaten als Ausgabedaten der optimierten trainierten Funktion,

    wobei die trainierte Funktion insbesondere eine optimierte trainierte Funktion, OTF, ist, die mit einem Verfahren nach einem der Ansprüche 1 bis 12 erzeugt wurde.

14. Systemsteuereinheit für eine Magnetresonanzvorrichtung, die ausgebildet ist, ein Verfahren nach Anspruch 13 durchzuführen.

15. Computerprogrammprodukt, welches ein Programm umfasst und direkt in einen Speicher einer programmierbaren Systemsteuereinheit einer Magnetresonanzvorrichtung ladbar ist, mit Programmmitteln, um ein Verfahren nach Anspruch 13 auszuführen, wenn das Programm in der Systemsteuereinheit der medizinischen Bildgebungsvorrichtung ausgeführt wird.

**FIG 1**

FIG 2

FIG 3

EP 4 249 934 A1

FIG 4

| | |
|---|---|
| | — S210 |

↓

| | |
|---|---|
| | — S220 |

↓

| | |
|---|---|
| | — S230 |

↓

| | |
|---|---|
| | — S240 |

Europäisches Patentamt

European Patent Office

Office européen des brevets

**EUROPÄISCHER RECHERCHENBERICHT**

Nummer der Anmeldung

EP 22 16 4397

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2021/018583 A1 (GUI DAWEI [US] ET AL) 21. Januar 2021 (2021-01-21) * Absatz [0091] – Absatz [0122] * * Abbildungen 4-6 * ----- | 1-5,7,8, 10,13-15 | INV. G01R33/24 G01R33/56 G01R33/565 |
| A | FUNAI A K ET AL: "Regularized field map estimation in MRI", IEEE TRANSACTIONS ON MEDICAL IMAGING, IEEE, USA, Bd. 27, Nr. 10, 1. Oktober 2008 (2008-10-01), Seiten 1484-1494, XP002657572, ISSN: 0278-0062, DOI: 10.1109/TMI.2008.923956 * Section II * ----- | 1-15 | |

| RECHERCHIERTE SACHGEBIETE (IPC) |
|---|
| G01R |

Der vorliegende Recherchenbericht wurde für alle Patentansprüche erstellt

| Recherchenort | Abschlußdatum der Recherche | Prüfer |
|---|---|---|
| München | 24. August 2022 | Streif, Jörg Ulrich |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**    EP 22 16 4397

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-08-2022

| Im Recherchenbericht angeführtes Patentdokument | Datum der Veröffentlichung | Mitglied(er) der Patentfamilie | Datum der Veröffentlichung |
|---|---|---|---|
| US 2021018583 A1 | 21-01-2021 | CN 112240995 A<br>US 2021018583 A1 | 19-01-2021<br>21-01-2021 |

Für nähere Einzelheiten zu diesem Anhang : siehe Amtsblatt des Europäischen Patentamts, Nr.12/82